# EUROPEAN PATENT APPLICATION

(11) **EP 4 471 059 A1**
(43) Date of publication of application: **04.12.2024**
(21) Application number: 22922665.9
(22) Date of filing: 26.01.2022
(51) Int. Cl.: C07K 16/22, A61K 39/395, A61P 35/00, C12N 15/13

(54) **ANTIBODY MOLECULE AGAINST GROWTH AND DIFFERENTIATION FACTOR 15 AND USE THEREOF**

(71) Applicant: Yunnan Baiyao Group Co., Ltd., Kunming, Yunnan 650500 (CN)
(72) Inventor: NING, Jinying, Kunming, Yunnan 650500 (CN); PENG, Hao, Kunming, Yunnan 650500 (CN); HAO, Feng, Kunming, Yunnan 650500 (CN); HE, Feng, Kunming, Yunnan 650500 (CN); WU, Guojin, Kunming, Yunnan 650500 (CN)
(74) Representative: Cabinet Laurent & Charras
(86) International application number: PCT/CN2022/074026
(87) International publication number: WO 2023/141815

(57) **Abstract**

Provided in the present disclosure is an antibody or fragment thereof against a human growth and differentiation factor 15 (GDF15). Further provided in the present disclosure is the use of the antibody or fragment thereof in the preparation of a drug for treating diseases or conditions. The antibody or fragment thereof provided in the present disclosure can bind to human GDF15 with high affinity and specificity, blocks the interaction of GDF15 with receptor GFRAL thereof, and has a longer half-life period in vivo in comparison with antibodies of the same kind.

## Description

### Technical Field

The present disclosure relates to the field of biomedicine, and in particular, to an antibody molecule and antigen-binding fragment thereof that specifically bind to growth differentiation factor 15 (GDF15), as well as use thereof.

### Background

Growth differentiation factor 15 (GDF15) is a member of the trans-forming growth factor β (TGFβ) superfamily. It is responsible for regulating food intake, energy expenditure and body weight in response to metabolic and toxin-induced stress in human. The receptor of GDF15 is the glial cell line derived neurotrophic factor (GDNF) family receptor α-like (GFRAL). Upon binding to its receptor GFRAL, GDF15 can activate neurons located in the area postrema of brainstem and nucleus of solitary tract, because these areas have expression of its receptor GFRAL. Then, GDF15 triggers the activation of neurons located in parabrachial nucleus and central amygdala, forming an eating response to stress conditions. Due to these functions, GDF15 has attracted people's attention as a potential target for the treatment of obesity and related metabolic diseases.

Circulating levels of GDF15 have long been associated with lower body mass index (BMI) and cachexia in patients with cancer, heart failure or chronic kidney disease. Recent data suggest that upon binding to neurons expressing GFRAL in brainstem and triggering the activation of neurons in parabrachial nucleus and central amygdala, GDF15 leads to appetite suppression and ultimately weight loss. Therefore, it has become a potential target for the treatment of cachexia-related weight loss and obesity.

### Summary

A technical problem to be solved by the present disclosure is to provide a novel antibody, such as a monoclonal antibody, which binds to human growth differentiation factor 15 (GDF15) with high affinity and specificity, for use as a new therapeutic agent in treating diseases or symptoms such as cachexia.

In view of the technical problem, the purpose of the present disclosure is to provide an anti-GDF15 antibody or fragment thereof, as well as uses thereof. "The fragment" of the antibody molecule described herein encompasses various functional fragments of the antibody, such as antigen-binding fragment of the antibody.

The present disclosure provides the following technical solutions.

In one aspect, the present disclosure provides an antibody or antigen-binding fragment thereof, which can specifically bind to GDF15, in particular human GDF15. In an embodiment, the antibody of the present disclosure is a murine antibody obtained by using the extracellular domain of human GDF15 as an immunogen, or a humanized antibody obtained based on the murine antibody.

Specifically, the present disclosure provides an antibody or fragment thereof, wherein the antibody or fragment thereof comprises a variable region of a heavy chain (VH) and a variable region of a light chain (VL), wherein the variable region of the heavy chain (VH) and the variable region of the light chain (VL) comprise a combination of complementary determining regions (CDRs) of H-CDR1, H-CDR2, H-CDR3, L-CDR1, L-CDR2 and L-CDR3 selected from the following:
(1) H-CDR1, H-CDR2 and H-CDR3 set forth in SEQ ID NO.23 (TSGMGVG), SEQ ID NO.24 (HII,WDDVKRYNPALKS) and SEQ ID NO.25 (MAWDWFAY), respectively, and L-CDR1, L-CDR2 and L-CDR3 set forth in SEQ ID NO.26 (KASQNVDTNVA), SEQ ID NO.27 (SASYRSS) and SEQ ID NO.28 (QQYHSYPT), respectively;
(2) H-CDR1, H-CDR2 and H-CDR3 set forth in SEQ ID NO.23 (TSGMGVG), SEQ ID NO.29 (HIRWDDVKRYNPALKS) and SEQ ID NO.25 (MAWDWFAY), respectively, and L-CDR1, L-CDR2 and L-CDR3 set forth in SEQ ID NO.30 (KASQNVDTDVA), SEQ ID NO.31 (SASYRYS) and SEQ ID NO.32 (HQYNSYPT), respectively;
(3) H-CDR1, H-CDR2 and H-CDR3 set forth in SEQ ID NO.33 (TAGMTVG), SEQ ID NO.34 (HIWWNDDKYYNPALKS) and SEQ ID NO.35 (IATMNYAMDY), respectively, and L-CDR1, L-CDR2 and L-CDR3 set forth in SEQ ID NO.36 (RASQSVSTSSFSYMH), SEQ ID NO.37 (YASNLES) and SEQ ID NO.38 (QHSWEIPYT), respectively;
(4) H-CDR1, H-CDR2 and H-CDR3 set forth in SEQ ID NO.39 (TSGMGVD), SEQ ID NO.40 (HIYWDDDKRYNPSLKS) and SEQ ID NO.41 (RAWDAMDY), respectively, and L-CDR1, L-CDR2 and L-CDR3 set forth in SEQ ID NO.42 (KARQNVGTNVA), SEQ ID NO.31 (SASYRYS) and SEQ ID NO.43 (QQYNSYPYT), respectively;
(5) H-CDR1, H-CDR2 and H-CDR3 set forth in SEQ ID NO.23 (TSGMGVG), SEQ ID NO.44 (HIWWNDDKYYNPSLKS) and SEQ ID NO.45 (GAYDFFDY), respectively, and, L-CDR1, L-CDR2 and L-CDR3 set forth in SEQ ID NO.26 (KASQNVDTNVA), SEQ ID NO.31 (SASYRYS) and SEQ ID NO.46 (QQYNTYPYT), respectively; and
(6) H-CDR1, H-CDR2 and H-CDR3 set forth in SEQ ID NO.47 (TSGIGIT), SEQ ID NO.48 (TIWWDDDNRYNPSLKS) and SEQ ID NO.49 (SAWDWFAY), respectively, and L-CDR1, L-CDR2 and L-CDR3 set forth in SEQ ID NO.50 (KASQNVGTNVA), SEQ ID NO.51 (SASYRNS) and SEQ ID NO.52 (QQYNSHPVT), respectively.

The antibody or fragment thereof provided by the present disclosure is an antibody or fragment thereof against GDF15, in particular human GDF15, capable of binding to said GDF15 with high affinity and specificity. The combination of light and heavy chain CDRs comprised in the antibody or fragment thereof is from a specific antibody of the present disclosure (see the Examples). Based on the amino acid sequence of the variable region comprised in said specific antibody, the CDRs comprised therein can be conventionally determined. According to an embodiment of the present disclosure, the CDRs in the amino acid sequence of the variable region of said specific antibody can be determined using Kabat or any other definition methods such as IMGT, ABM, Chotia, etc. Therefore, the CDRs of the light and heavy chains and their combinations determined by other methods known in the art are also within the scope of the present disclosure.

Preferably, in the antibody or fragment thereof provided by the present disclosure, the variable region of the heavy chain may comprise an amino acid sequence set forth in or an amino acid sequence having at least 75% identity with SEQ ID NO: 7, SEQ ID NO: 9, SEQ ID NO: 11, SEQ ID NO: 13, SEQ ID NO: 15, SEQ ID NO: 17, SEQ ID NO: 19 or SEQ ID NO: 21; and/or, the variable region of the light chain may comprise an amino acid sequence set forth in or an amino acid sequence having at least 75% identity with SEQ ID NO: 8, SEQ ID NO: 10, SEQ ID NO: 12 , SEQ ID NO: 14, SEQ ID NO: 16, SEQ ID NO: 18, SEQ ID NO: 20 or SEQ ID NO: 22. As used herein relevant to sequences, "at least 75% identity" means any identity percentage of 75% and more than 75%, such as, at least 80%, preferably at least 85%, more preferably at least 90%, further preferably at least 91%, 92%, 93 %, 94%, 95%, 96%, 97%, 98% or even 99% identity, etc.

Further preferably, in the antibody or fragment thereof provided by the present disclosure, the variable region of the heavy chain and the variable region of the light chain respectively comprise:
(1) an amino acid sequence set forth in or an amino acid sequence having at least 75% identity with SEQ ID NO: 7, and an amino acid sequence set forth in or an amino acid sequence having at least 75% identity with SEQ ID NO: 8;
(2) an amino acid sequence set forth in or an amino acid sequence having at least 75% identity with SEQ ID NO: 9, and an amino acid sequence set forth in or an amino acid sequence having at least 75% identity with SEQ ID NO: 10;
(3) an amino acid sequence set forth in or an amino acid sequence having at least 75% identity with SEQ ID NO: 11, and an amino acid sequence set forth in or an amino acid sequence having at least 75% identity with SEQ ID NO: 12;
(4) an amino acid sequence set forth in or an amino acid sequence having at least 75% identity with SEQ ID NO: 13, and an amino acid sequence set forth in or an amino acid sequence having at least 75% identity with SEQ ID NO: 14;
(5) an amino acid sequence set forth in or an amino acid sequence having at least 75% identity with SEQ ID NO: 15, and an amino acid sequence set forth in or an amino acid sequence having at least 75% identity with SEQ ID NO: 16;
(6) an amino acid sequence set forth in or an amino acid sequence having at least 75% identity with SEQ ID NO: 17, and an amino acid sequence set forth in or an amino acid sequence having at least 75% identity with SEQ ID NO: 18;
(7) an amino acid sequence set forth in or an amino acid sequence having at least 75% identity with SEQ ID NO: 19, and an amino acid sequence set forth in or an amino acid sequence having at least 75% identity with SEQ ID NO: 20;
(8) an amino acid sequence set forth in or an amino acid sequence having at least 75% identity with SEQ ID NO: 21, and an amino acid sequence set forth in or an amino acid sequence having at least 75% identity with SEQ ID NO: 22.

In particular, the antibody or fragment thereof of the present disclosure comprises at least a variable region of a heavy chain and a variable region of a light chain, both of which comprise the CDRs described above and the framework regions in between, and these domains are arranged as: FR1-CDR1-FR2-CDR2-FR3-CDR3-FR4. Moreover, optionally, the at most 25% difference in an amino acid sequence caused by "at least 75% identity" may exist in any framework region in the variable region of the heavy chain or the variable region of the light chain, or in any domain or sequence in the antibody or fragment thereof of the present disclosure other than the variable region of the heavy chain and the variable region of the light chain. The difference may be caused by deletion, addition or substitution of an amino acid at any position.

The antibody provided by the present disclosure is a murine antibody, a chimeric antibody or a fully or partially humanized antibody that binds to GDF15, in particular human GDF15; the fragment is a semi-antibody or an antigen-binding fragment of the antibody, including scFv, dsFv, (dsFv)₂, Fab, Fab', F(ab')₂ or Fv fragment. Preferably, the antibody is a monoclonal antibody or a single-chain antibody.

In addition to the variable region, the antibody or fragment thereof also comprises a human or murine constant region, preferably a human or murine constant region of a heavy chain (CH) and/or a human or murine constant region of a light chain (CL); preferably, the antibody or fragment thereof comprises a heavy chain and a light chain; more preferably, the antibody or fragment thereof comprises a constant region of a heavy chain selected from IgG, IgA, IgM, IgD or IgE and/or a constant region of a kappa or lambda light chain. According to an embodiment of the present disclosure, the antibody is a monoclonal antibody, preferably a murine, chimeric or humanized monoclonal antibody. According to an embodiment of the present disclosure, the monoclonal antibody is of IgG, particularly IgG1.

Further preferably, the antibody or fragment thereof provided by the present disclosure comprises a constant region of a heavy chain comprising an amino acid sequence set forth in or an amino acid sequence having at least 75% identity with SEQ ID NO: 3; and/or a constant region of a light chain comprising an amino acid sequence set forth in or an amino acid sequence having at least 75% identity with SEQ ID NO: 4. Alternatively, it comprises a constant region of a heavy chain comprising an amino acid sequence set forth in or an amino acid sequence having at least 75% identity with SEQ ID NO: 5; and/or a constant region of a light chain comprising an amino acid sequence set forth in or an amino acid sequence having at least 75% identity with SEQ ID NO: 6.

On the other hand, the present disclosure also provides a nucleic acid molecule comprising a nucleotide sequence encoding the antibody or fragment thereof described in the present disclosure, or comprising a nucleotide sequence encoding a CDR of a heavy chain, a CDR of a light chain, a variable region of a light chain, a variable region of a heavy chain, a heavy chain or a light chain comprised in the antibody or fragment thereof.

In another aspect, the present disclosure provides a vector comprising the nucleic acid molecule as described above. The vector may be a eukaryotic expression vector, a prokaryotic expression vector, an artificial chromosome, a phage vector, and the like.

In another aspect, the present disclosure provides a host cell comprising the nucleic acid molecule or vector as described above, or a host cell transformed or transfected by the nucleic acid molecule or vector as described above. The host cell may be any prokaryotic or eukaryotic cell, such as a bacterial or insect, fungus, plant or animal cell.

The antibody or fragment thereof provided by the present disclosure may be obtained by any method known in the art. For example, the host cell is cultivated in a condition where the host cell is allowed to express a variable region of a heavy chain and/or a variable region of a light chain or a heavy chain and a light chain of the antibody to construct the antibody. Optionally, the method also includes the step of recovering the antibody produced.

On the other hand, the present disclosure also provides a composition comprising the antibody or fragment thereof, the nucleic acid molecule, the vector and/or the host cell of the present disclosure. Preferably, the composition is a pharmaceutical composition, which optionally further comprises a pharmaceutically acceptable carrier, adjuvant or excipient.

The antibody or fragment thereof, the nucleic acid molecule, the vector, the host cell and/or the composition provided by the present disclosure has one or more activities of increasing the subject's appetite, food intake, body weight, muscle mass, etc., and also has anti-tumor effects. Therefore, the present disclosure also provides the following technical solutions.

In another aspect, the present disclosure also provides use of the antibody or fragment thereof, the nucleic acid molecule, the vector, the host cell and/or the composition in the manufacture of a medicament for treating a disease or condition, wherein the disease or condition is mediated by or associated with the expression of GDF15; and/or the disease or condition is tumor (e.g., cancer), heart failure, chronic kidney disease, anorexia, sarcopenia or cachexia.

Further, the present disclosure also provides a method for treating a disease or condition comprising administering the antibody or fragment thereof, the nucleic acid molecule, the vector, the host cell and/or the composition provided by the present disclosure to a subject in need thereof, wherein the disease or condition is mediated by or associated with the expression of GDF15; and/or the disease or condition is tumor (e.g., cancer), heart failure, chronic kidney disease, anorexia, sarcopenia or cachexia.

Preferably, the subject is mammal; more preferably, the subject is human.

The present disclosure also provides a method for detecting or diagnosing a disease or condition comprising contacting the antibody molecule or fragment thereof, the nucleic acid molecule, the vector, the host cell and/or the composition of the present disclosure with a sample from a subject, wherein the disease or condition is mediated by or associated with the expression of GDF15; and/or the disease or condition is tumor (e.g., cancer), heart failure, chronic kidney disease, anorexia, sarcopenia or cachexia. Preferably, the subject is mammal; more preferably, the subject is human.

In addition, the present disclosure provides a kit comprising the antibody or fragment thereof, the nucleic acid molecule, the vector, the host cell and/or the composition. The kit is used for the treatment or detection or diagnosis as described above. Optionally, the kit may also include instructions for use.

The present disclosure provides a novel antibody against human GDF15 capable of binding to human GDF15 with high affinity and specificity, and blocking the interaction between GDF15 and its receptor GFRAL. Experiments have shown that the antibody provided by the present disclosure has higher GDF15 affinity and specificity, longer *in vivo* half-life, and better blocking effect compared to other known antibodies from the same type.

### BRIEF DESCRIPTION OF THE DRAWINGS

The embodiments of the present disclosure are described in detail below with reference to the drawings, wherein:
Figure 1 shows the results of the ELISA assay indicating the hybridoma cell culture supernatant binds to human GDF15.
Figure 2 shows the results of the ELISA assay indicating the hybridoma cell culture supernatant binds to monkey GDF15.
Figure 3 shows the results of the ELISA assay indicating the hybridoma cell culture supernatant blocks the binding of GDF15 to its receptor GFRAL.
Figure 4 shows the results of the ELISA assay indicating the antibody binds to human GDF15.
Figure 5 shows the results of the ELISA assay indicating the antibody binds to monkey GDF15.
Figure 6 shows the results of the ELISA assay indicating the antibody blocks the binding of GDF15 to its receptor GFRAL.
Figure 7 shows the results of the ELISA assay indicating the antibody binds to human GDF15 after being placed in PBS and plasma.
Figure 8 shows the results of the competitive ELISA assay indicating the antibody blocks the binding of GDF15 to its receptor GFRAL.
Figure 9 shows the results of blocking the function of GDF15 by the antibody detected on reporter cells expressing GFRAL.
Figure 10 shows the results of neutralizing GDF15 by the antibody detected on reporter cells expressing GFRAL; wherein 10-1: Ponsegromab; 10-2: 53E5(hz).
Figure 11 shows the results of the binding specificity of the antibody to GDF15, GDF1 and GDF3 .
Figure 12 shows the results of the binding specificity of the antibody to PBMCs.
Figure 13 shows the PK experiment results of the antibody in mice; 13-1: AV380-hIgG1; 13-2: Ponsegromab; 13-3: 53E5(hz).
Figure 14 shows the effects of inhibiting cachexia by the antibody tested in mice.
Figure 15 shows the effects of neutralizing GDF15 by the antibody tested in mice.

### Examples

The present disclosure is illustrated below with reference to specific examples. It will be understood that these examples are only used to illustrate the present disclosure and are not intended to limit the scope of the present disclosure in any way.

The experimental methods in the following examples are conventional unless otherwise specified. The raw materials, reagents, etc. used in the following examples are commercially available products unless otherwise specified.

### Example 1: Preparation of hybridoma cells

A fusion protein comprising the extracellular region of GDF15 protein (Genbank accession number NM_004864.3) and mouse IgG2a-FC (Genbank accession number AAH31470.1) was used as an immunogen to immunize mice. The Quick Antibody-Mouse 5W water-soluble adjuvant was used as adjuvant. The titers were measured 2 weeks after booster immunization, and mice with high titers were selected for boost immunization. The mouse serum was collected after 3 days, and the spleens were obtained via dissection and the spleen cells were isolated.

The spleen cells were fused with cultured myeloma cells and plated onto 96-well plate, and selective culture medium was added simultaneously for screening. The culture medium was replaced after 7 days. The ELISA assay was performed after 10 days, and the cells with OD values more than 10 times greater than that of the negative control were selected for flow cytometry.

Double-positive cells were selected and sub-clones were plated by the limited dilution method. Monoclonal cells were selected. Supernatants of the selected monoclonal cells were taken for ELISA and flow cytometry. Double-positive cells were selected for culture expansion. Cell supernatants were purified by Protein G gravity column affinity chromatography to obtain hybridoma antibodies.

### Example 2: Binding of hybridoma antibodies to human GDF15 detected by ELISA

A fusion protein comprising the extracellular region of human GDF15 protein (Genbank accession number NM_004864.3) and human IgG1-FC (Genbank accession number CAC20454.1 ) was diluted to 1 µg/ml with coating solution, followed by adding to ELISA plate at 50 µl/well and incubating at 4°C overnight. The solution in the wells was discarded and the plate was washed 3 times with washing solution, each time for 3-5 minutes, and dried by tapping. 200 µl of blocking solution was added to each well and incubated at 4°C overnight. The plate was washed 3 times with washing solution, and the coated plate may be stored at -20°C or 4°C for future use.

The antibodies to be tested (purified antibodies from hybridoma, see Table 1), positive control antibody AV380-mIgG1 (its sequence is from Aveo Oncology; see SEQ ID NO.1 and SEQ ID NO.2 for its heavy chain and light chain variable regions, respectively; see SEQ ID NO.3 and SEQ ID NO.4 for its heavy chain and light chain constant regions, respectively) and blank control (PBS) were added to the wells. Antibodies had a start concentration of 20 µg/mL, 3.16-fold dilution, and 10 gradients in total. The wells were incubated at 37°C for 1 hour, washed, and dried via tapping. Then the enzyme-labeled secondary antibody, horseradish peroxidase-labeled goat anti-mouse IgG (SIGMA, catalog number A9044-2ml) was added to each well, at a dilution of 1:10000, 100 µl/well. The wells were incubated at 37°C for 2 hours, washed, and dried via tapping. 100 µl of freshly prepared developing solution TMB was added to each well and incubated at 37°C for 30 minutes.

The sequences of AV 380 -m IgG1 are as follows:
Heavy chain variable region VH (SEQ ID NO.1): 
EVLLQQSGPELVKPGASVKIPCKASGYTFTDYNMDWVKQSHGKSLEWIGQINPNNGGI
FFNQKFKGKATLTVDKSSNTAFMEVRSLTSEDTAVYYCAREAITTVGAMDYWGQGTS
VTVSS
Light chain variable region VL (SEQ ID NO.2): 
DIQMTQSPASLSASVGETVTITCRTSENLHNYLAWYQQKQGKSPQLLVYDAKTLADGV
PSRFSGSGSGTQYSLKINSLQPEDFGSYYCQHFWSSPYTFGGGTKLEIK
Heavy chain constant region (SEQ ID NO.3): 
AKTTPPSVYPLAPGSAAQTNSMVTLGCLVKGYFPEPVTVTWNSGSLSSGVHTFPAVLQ
SDLYTLSSSVTVPSSTWPSETVTCNVAHPASSTKVDKKIVPRDCGCKPCICTVPEVSSVFI
FPPKPKDVLTITLTPKVTCVVVDISKDDPEVQFSWFVDDVEVHTAQTQPREEQFNSTFR
SVSELPIMHQDWLNGKEFKCRVNSAAFPAPIEKTISKTKGRPKAPQVYTIPPPKEQMAK
DKVSLTCMITDFFPEDITVEWQWNGQPAENYKNTQPIMDTDGSYFVYSKLNVQKSNW
EAGNTFTCSVLHEGLHNHHTEKSLSHSPGK
Light chain constant region (SEQ ID NO.4):

RADAAPTVSIFPPSSEQLTSGGASVVCFLNNFYPKDINVKWKIDGSERQNGVLNSWTD
QDSKDSTYSMSSTLTLTKDEYERHNSYTCEATHKTSTSPIVKSFNRNEC

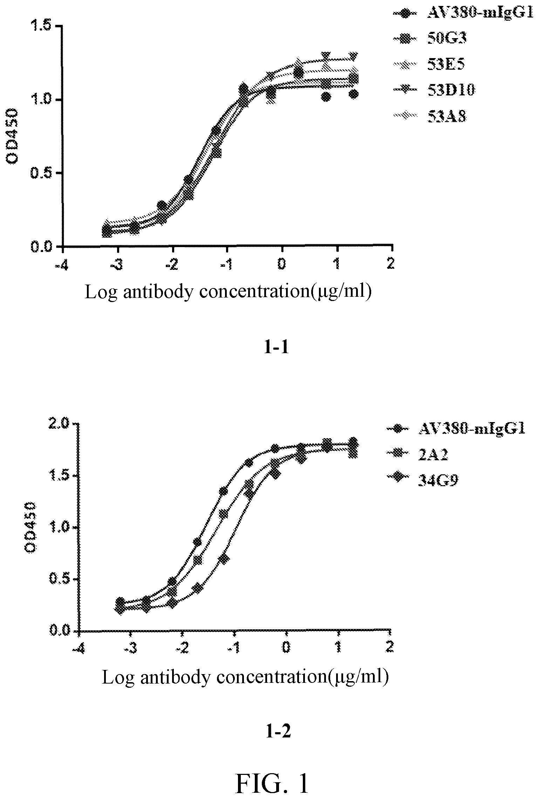

2 mol/L H₂SO₄ was added to terminate the reaction, and the OD values were read on enzyme-linked immunosorbent reader. The results were shown in Table 1-1, Table 1-2 and Figure 1-1 and 1-2.

**Table 1-1: ELISA results of hybridoma antibodies binding to human GDF15**

| Sample | EC50 (µg/ml) |
|---|---|
| AV380-mIgG1 | 0.03134 |
| 50G3 | 0.05563 |
| 53E5 | 0.04546 |
| 53D10 | 0.06025 |
| 53A8 | 0.03780 |

**Table 1-2: ELISA results of hybridoma antibodies binding to human GDF15**

| Sample | EC50 (µg/ml) |
|---|---|
| AV380-mIgG1 | 0.02958 |
| 2A2 | 0.04602 |
| 34G9 | 0.1094 |

### Example 3: Binding of hybridoma antibodies to monkey GDF15 (cynoGDF15) detected by ELISA

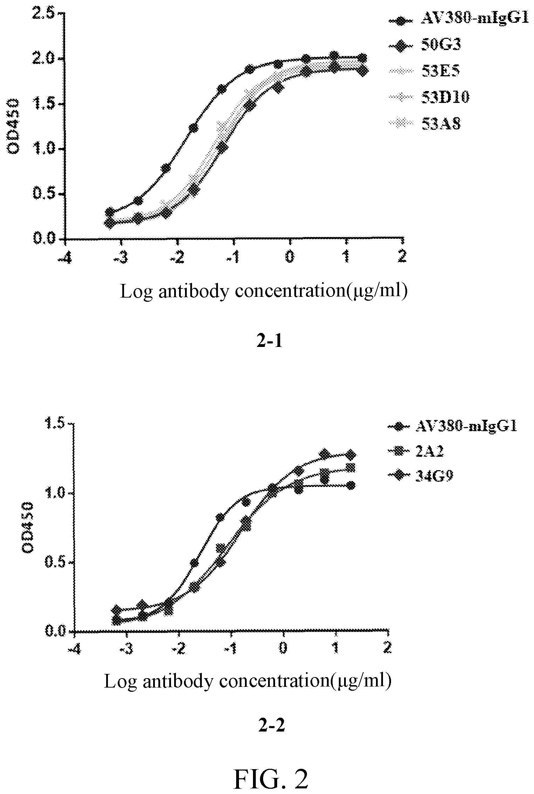

The experiment was performed as detailed in Example 2, except that the fusion protein in Example 2 was replaced with a fusion protein comprising the extracellular region of monkey GDF15 protein (Genbank accession number EHH29815.1) and human IgG1-FC (Genbank accession number CAC20454.1). The results were shown in Table 2-1, Table 2-2 and Fig.2-1 and 2-2.

**Table 2-1: ELISA results of hybridoma antibodies binding to monkey GDF15**

| Sample | EC50 (µg/ml) |
|---|---|
| AV380-mIgG1 | 0.01459 |
| 50G3 | 0.07697 |
| 53E5 | 0.04539 |
| 53D10 | 0.05550 |
| 53A8 | 0.06765 |

**Table 2-2: ELISA results of hybridoma antibodies binding to monkey GDF15**

| Sample | EC50 (µg/ml) |
|---|---|
| AV380-mIgG1 | 0.02577 |
| 2A2 | 0.08271 |
| 34G9 | 0.1574 |

### Example 4: Blocking of the binding of GDF15 to its receptor GFRAL by Hybridoma antibodies detected with ELISA

A fusion protein comprising the extracellular region of GFRAL protein (Genbank accession number NM_207410.2) and human IgG1-FC (Genbank accession number CAC20454.1) was diluted to 1 µg/ml with coating solution, followed by adding to ELISA plate at 50 µl/well and incubating at 4°C overnight. The solution in the wells was discarded and the plate was washed 3 times with washing solution, each time for 3-5 minutes, and dried via tapping. 200 µl of blocking solution was added to each well followed by incubating at 4°C overnight. The plate was washed 3 times with washing solution, and the coated plate may be stored at -20°C or 4°C for future use.

The antibodies to be tested, positive control antibody AV380-mIgG1 and blank control (PBS) were added to the wells. Antibodies had a start concentration of 20 µg/mL, 3.16-fold dilution, and 10 gradients in total. At the same time, GDF15-mFC protein (GDF15: Genbank accession number NM_004864.3; mFC: Genbank accession number AAH31470.1) was added at 20 ng/well and mixed with the antibodies to be tested. The wells were incubated at 37°C for 1 hour, washed, and dried via tapping. Then the enzyme-labeled secondary antibody, horseradish peroxidase-labeled goat anti-mouse IgG (SIGMA, catalog number A9044-2ml) was added to each well, at a dilution of 1: 10000, 50-100 µl/well. The wells were incubated at 37°C for 2 hours, washed, and dried via tapping. 100 µl of freshly prepared developing solution TMB was added to each well and incubated at 37°C for 30 minutes. 2 mol/L H₂SO₄ was added to terminate the reaction, and the OD value was obtained on an enzyme-linked immunosorbent reader.

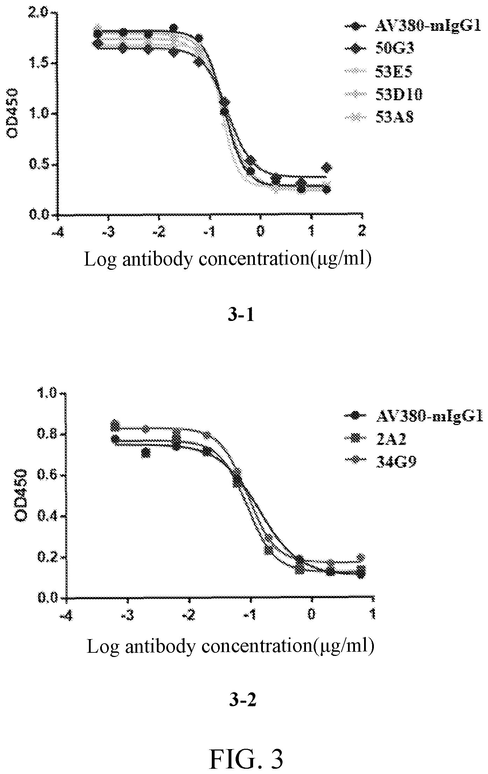

The results were shown in Table 3-1, Table 3-2 and Fig. 3-1 and 3-2.

**Table 3-1: ELISA results of hybridoma antibodies blocking the binding of GDF15 to its receptor GFRAL**

| Sample | IC50 (µg/ml) |
|---|---|
| AV380-mIgG1 | 0.1977 |
| 50G3 | 0.2318 |
| 53E5 | 0.1862 |
| 53D10 | 0.2126 |
| 53A8 | 0.2046 |

**Table 3-2: ELISA results of hybridoma antibodies blocking the binding of GDF15 to its receptor GFRAL**

| Samples (shown as hybridoma cell number) | IC50 (µg/ml) |
|---|---|
| AV380-mIgG1 | 0.1395 |
| 2A2 | 0.08984 |
| 34G9 | 0.09157 |

The variable region sequences of the above murine antibodies are as follows (wherein the heavy chain and light chain CDRs according to the Kabat definition method are shown in bold and underlined):

### A1 (murine anti-53E5)

>53E5-H (VH/HCDR-1/HCDR-2/HCDR-3: SEQ ID NO.7/23/24/25) 
QVQLKESGPGILQPSQTLSLTCSFSGFSLSTSGMGVGWIRQPSGKGLEWLAHILWDDV
KRYNPALKSRLTISKDSSNSQIFLKIASVDTADTATYYCVQMAWDWFAYWGQGTLV
TVSA
>53E5-L (VL/LCDR-1/LCDR-2/LCDR-3: SEQ ID NO.8/26/27/28)

DIVMTQAQKFMSTSVEDRVSVTCKASQNVDTNVAWYQQKPGQSPKALIYSASYRSS
GVPDRFTGSGSGTDFTLTISNVQSEDLAEYFCQQYHSYPTFGGGTKLEIK

### A2 (murine anti-53A8)

>53A8-H (VH/HCDR-1/HCDR-2/HCDR-3: SEQ ID NO.9/23/29/25) 
QVQLKESGPGILQPSQTLSLTCSFSGFSLSTSGMGVGWIRQPSGKGLEWLAHIRWDDV
KRYNPALKSRLTISKDTSSSQIFLKIASVDTADTATYYCAQMAWDWFAYWGQGTLV
TVSA
>53A8-L (VL/LCDR-1/LCDR-2/LCDR-3: SEQ ID NO.10/30/31/32)

DIVMTQAQKFMSTSVGDRVSVTCKASQNVDTDVAWYQQKPGQSPKALIYSASYRYS
GVPDRFTGSGSGTDFTLTITNVQSEDLAEYFCHQYNSYPTFGGGTKLEIK

### A3 (murine anti-34G9)

>34G9-H (VH/HCDR-1/HCDR-2/HCDR-3: SEQ ID NO.11/33/34/35) 
QVQLKESGPGILQPSQTLSLTCSFSGFSLNTAGMTVGWIRQPSGKGLEWLAHIWWND
DKYYNPALKSRLTISKDTSNNQIFLEIASVVTADTATYYCTRIATMNYAMDYWGQGT
SVTVSS
>34G9-L (VL/LCDR-1/LCDR-2/LCDR-3: SEQ ID NO.12/36/37/38)

DIVMTQAPASLAVSLGQRATISCRASQSVSTSSFSYMHWYQQKPGQPPKLLIKYASNL
ESGVPARFSGSGSGTDFTLNIHPVEEEDTATYYCQHSWEIPYTFGGGTKLEIK

### A4 (murine anti-2A2)

>2A2-H (VH/HCDR-1/HCDR-2/HCDR-3: SEQ ID NO.13/39/40/41) 
QVTLKESGPGILQPSQTLSLTCSFSGFSLTTSGMGVDWIRQSSGKGLEWLAHIYWDDD
KRYNPSLKSRLTISKDTSSNQVFLKITSVDTADTATYYCARRAWDAMDYWGQGTSF
TVSS
>2A2-L (VL/LCDR-1/LCDR-2/LCDR-3: SEQ ID NO.14/42/31/43)

DIVMTQAQKFMSTSVGDRVSVTCKARQNVGTNVAWYQQKPGQSPKALIYSASYRYS
GVPDRFTGSGSGTDFTLTISNVQSEDLAEYFCQQYNSYPYTFGGGTKLEIK

### A5 (murine anti-50G3)

>50G3-H (VH/HCDR-1/HCDR-2/HCDR-3: SEQ ID NO.15/23/44/45) 
QVTLKESGPGILKPSQTLSLTCSFSGFSLSTSGMGVGWIRQPSGKGLEWLAHIWWND
DKYYNPSLKSHLTISKDTSRNQVFLMITSVDTADTATYYCARGAYDFFDYWGQGTTL
TVSS
>50G3-L (VL/LCDR-1/LCDR-2/LCDR-3: SEQ ID NO.16/26/31/46)

DIVMTQAQNFMSTSVGDRVSVTCKASQNVDTNVAWYQQKPGQSPKALIYSASYRYS
GVPDRFTGSGSGTDFTLTISNVQSDDLADYFCQQYNTYPYTFGGGTKLEIK

### A6 (murine anti-53D10)

>53D10-H (VH/HCDR-1/HCDR-2/HCDR-3: SEQ ID NO.17/47/48/49) 
QVTLKESGPGIVQPSQPFRLTCTFSGFSLSTSGIGITWIRQPSGKGLEWLATIWWDDDN
RYNPSLKSRLTVSKDTSNNQAFLNIITVETADTAIYYCVQSAWDWFAYWGQGTLVTV
SA
>53D10-L (VL/LCDR-1/LCDR-2/LCDR-3: SEQ ID NO.18/50/51/52)

DIVMTQSQKFMSTSVGDRVSVTCKASQNVGTNVAWYQQKSGQSPKALIYSASYRNS
GVPDRFTGSGSGTDFTLTISNVESEDLAEYFCQQYNSHPVTFGGGTKLEIK

### Example 5: Affinity of murine antibodies binding to human GDF15

### Experimental procedure:

1. Choose Capture Surface Dip of the same species;
2. Take a 96-well plate, add 200 µl SD buffer (1×PBS + 0.02% Tween20 + 0.1% BSA) to each well, place the plate in ForteBio Octet for pre-circulation;
3. Immobilize the antibody at a concentration of 10 µg/ml;
4. Dilute GDF15 (ACROBiosystems, GD5-H5149) into 6 gradients as 200nm, 100nm, 50nm, 25nm, 12.5nm, and 6.25nm, and add into the corresponding wells;
5. Test on the machine.

The results were shown in Table 4.

**Table 4: Affinity results of murine antibodies binding to human GDF15**

| Antibody | KD(M) | kon(1/Ms) | kdis(1/s) |
|---|---|---|---|
| AV380-mIgG1 | 9.70E-11 | 1.20E+06 | 1.17E-04 |
| 50G3 | 1.62E-10 | 2.02E+06 | 3.28E-04 |
| 53E5 | 8.50E-11 | 2.29E+06 | 1.94E-04 |
| 53D10 | 9.72E-11 | 2.24E+06 | 2.17E-04 |
| 53A8 | 1.40E-10 | 2.28E+06 | 3.18E-04 |
| 2A2 | 1.46E-10 | 1.47E+06 | 2.14E-04 |
| 34G9 | 8.95E-11 | 8.05E+05 | 7.20E-05 |

### Example 6: Humanization of murine antibodies

For A1 (murine anti-53E5), human-derived sequences IGHV4-39 and IGKV1-39 were selected as templates for heavy chain and light chain humanization, respectively. Homology modeling was performed on A1 monoclonal antibody, and structural simulation was performed to the Fab region. Homology modeling calculations were performed to finally obtain the predicted Fab structure of A1.

After comparing and analyzing the predicted Fab structure and heavy chain with the IGHV4-39 sequence, all murine amino acids were replaced with the corresponding human amino acids in IGHV4-39 template, except for the CDR regions and 2V, 24F, 50L, 51A, 69L, 73K, 75S, 78S, 80I, 98V, and 99Q in the VH which retained the original murine amino acids.

After comparing the predicted Fab structure and light chain with the IGKV1-39 sequence, all murine amino acids were replaced with the corresponding human amino acids in IGKV1-39 template, except for the CDR regions and 42Q, 43S, and 46A in the VL which retained the original murine amino acids.

The obtained humanized sequences are as follows (wherein the heavy chain and light chain CDRs according to the KABAT definition method are shown in bold and underlined):
>53E5-H humanized sequence (VH/HCDR-1/HCDR-2/HCDR-3: SEQ ID NO. 19/23/24/25) 
QVQLQESGPGLVKPSETLSLTCTFSGFSLSTSGMGVGWIRQPPGKGLEWLAHILWDD
VKRYNPALKSRLTISKDSSKSQISLKLSSVTAADTAVYYCVQMAWDWFAYWGQGTT
VTVSS
>53E5-L humanized sequence (VL/LCDR-1/LCDR-2/LCDR-3: SEQ ID NO.20/26/27/28)

DIQMTQSPSSLSASVGDRVTITCKASQNVDTNVAWYQQKPGQSPKALIYSASYRSSGV
PSRFSGSGSGTDFTLTISSLQPEDFATYYCQQYHSYPTFGQGTRLEIK

For A2 (murine antibody 53A8), human-derived sequences IGHV4-39 and IGKV1-39 were selected as templates for heavy chain and light chain humanization, respectively. Homology modeling was performed on A2 monoclonal antibody, and structural simulation was performed to the Fab region. Homology modeling calculations were performed to finally obtain the predicted Fab structure of A2.

After comparing and analyzing the predicted Fab structure and heavy chain with the IGHV4-39 sequence, all murine amino acids were replaced with the corresponding human amino acids in IGHV4-39 template, except for the CDR regions and 2V, 24F, 50L, 51A, 69L, 73K, 78S, 80I, and 99Q in the VH which retained the original murine amino acids.

After comparing and analyzing the predicted Fab structure and light chain with the IGKV1-39 sequence, all other murine amino acids were replaced with the corresponding human amino acids in IGKV1-39 template, except for the CDR regions and 42Q, 43S, and 46A in the VL which retained the original murine amino acids.

The obtained humanized sequences are as follows (wherein the heavy chain and light chain CDRs according to the KABAT definition method are shown in bold and underlined):
>53A8-H humanized sequence (VH/HCDR-1/HCDR-2/HCDR-3: SEQ ID NO.21/23/29/25) 
QVQLQESGPGLVKPSETLSLTCTFSGFSLSTSGMGVGWIRQPPGKGLEWLAHIRWDD
VKRYNPALKSRLTISKDTSKSQISLKLSSVTAADTAVYYCAQMAWDWFAYWGQGTT
VTVSS
>53A8-L humanized sequence (VL/LCDR-1/LCDR-2/LCDR-3: SEQ ID NO.22/30/31/32)

DIQMTQSPSSLSASVGDRVTITCKASQNVDTDVAWYQQKPGQSPKALIYSASYRYSGV
PSRFSGSGSGTDFTLTISSLQPEDFATYYCHQYNSYPTFGQGTRLEIK

The sequence set forth in SEQ ID NO.5 was served as the heavy chain constant region and the sequence set forth in SEQ ID NO.6 was served as the light chain constant region. The primers were designed for the humanized sequences in order to synthesize the corresponding sequences encoding the antibody heavy chain and light chain. The synthesized sequences were linked into eukaryotic expression vector. The obtained recombinant vector was transformed into *Escherichia coli* competent cells. The cells were cultured at 37°C overnight, and the monoclonal strains were picked for sequencing. The strains with the correct sequence were selected to obtain the recombinant vectors, which were transfected into mammalian expression cells 293F. The cells were cultured at 37°C and 5% CO₂ for 7 days. The supernatant was collected and purified to obtain the humanized antibody, which was named as "abbreviation of murine antibody name (hz)".
>CH1-CH3 heavy chain constant region (SEQ ID NO.5)
>CL1 light chain constant region (SEQ ID NO.6)

### Example 7: Binding of humanized antibodies to human GDF15 detected by ELISA

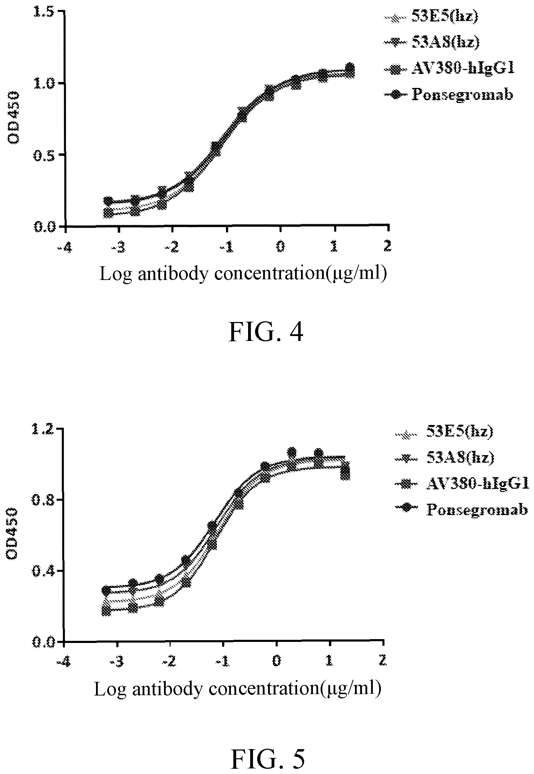

The experiment was performed as detailed in Example 2, except that the coating protein was GDF15-HIS (ACROBiosystems, CAT#: GD5-H5149), and the antibodies to be tested (the humanized antibodies), negative control antibody (hIgG1), positive control antibodies Ponsegromab (Pfizer; Drug Bank: D11909) and AV380-hIgG1 (the heavy chain and light chain constant regions of AV380-mIgG1 were replaced with the constant regions of human IgG, i.e., SEQ ID NO.5 and SEQ ID NO.6) were added to each well of the coated plate. The results were shown in Table 5 and Figure 4.

**Table 5: ELISA results of the humanized antibodies binding to human GDF15**

| Sample | EC50 (µg/ml) | OD450.Max |
|---|---|---|
| 53E5(hz) | 0.08331 | 1.053 |
| 53A8(hz) | 0.08266 | 1.051 |
| AV380-hIgG1 | 0.08233 | 1.066 |
| Ponsegromab | 0.09599 | 1.100 |

### Example 8: Binding of humanized antibodies to monkey GDF15 (cynoGDF15) detected by ELISA

The experiment was performed as detailed in Example 2, except that the coating protein was cynoGDF15-mFC (GDF15: Genbank accession number EHH29815.1; mFC: Genbank accession number AAH31470.1), and the antibodies to be tested (the humanized antibodies), negative control antibody (hIgG1), and positive control antibodies Ponsegromab and A V3 80-hIgG 1 were added to each well of the coated plate. The results were shown in Table 6 and Figure 5.

**Table 6: ELISA results of humanized antibodies binding to monkey GDF15**

| Sample | EC50 (µg/ml) | OD450.Max |
|---|---|---|
| 53E5(hz) | 0.08159 | 1.032 |
| 53A8(hz) | 0.07749 | 1.03 |
| AV380-hIgG1 | 0.07407 | 1.000 |
| Ponsegromab | 0.06973 | 1.067 |

### Example 9: Blocking of the binding of GDF15 protein to its receptor GFRAL by humanized antibodies

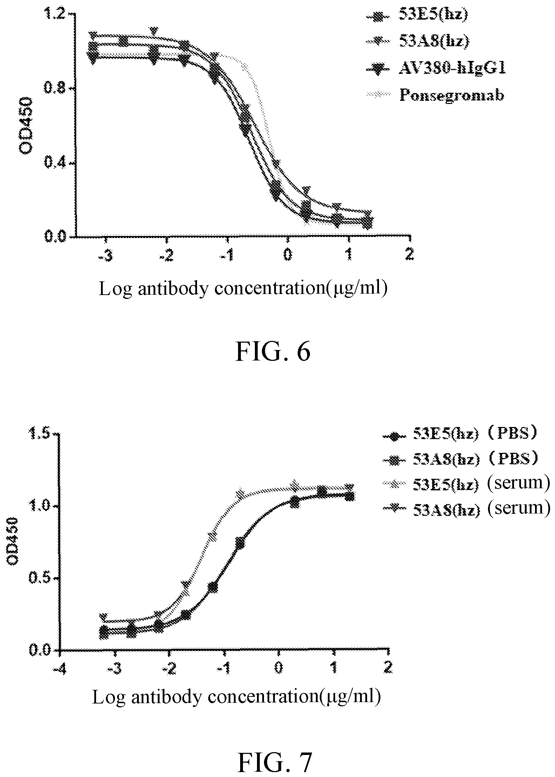

The experiment was performed as detailed in Example 4, except that the antibodies to be tested (the humanized antibodies), negative control antibody (hIgG1), and positive control antibodies Ponsegromab and AV380-hIgG1 were added to each well of the coated plate. The results were shown in Table 7 and Figure 6.

**Table 7: ELISA results of the humanized antibodies blocking the binding of GDF15 to its receptor GFRAL**

| Sample | IC50 (µg/ml) | OD450.Max |
|---|---|---|
| 53E5(hz) | 0.2525 | 1.049 |
| 53A8(hz) | 0.2831 | 1.104 |
| AV380-hIgG1 | 0.2311 | 10.965 |
| Ponsegromab | 0.5037 | 1.019 |

### Example 10: Affinity detection of humanized antibodies binding to human GDF15

The experiment was performed as detailed in Example 5, except that the GDF15 antibody was replaced with the antibodies to be tested (the humanized antibodies), negative control antibody (hIgG1), and positive control antibodies Ponsegromab and AV380-hIgG1. The results were shown in Table 8.

**Table 8: Affinity test results of humanized antibodies binding to human GDF15**

| Antibody | KD(M) | kon(1/Ms) | kdis(1/s) |
|---|---|---|---|
| 53E5(hz) | 2.93E-10 | 9.23E+05 | 2.71E-04 |
| 53A8(hz) | 2.66E-10 | 9.67E+05 | 2.57E-04 |
| AV380-hIgG1 | 8.45E-11 | 8.17E+05 | 6.91E-05 |
| Ponsegromab | <1.0E-12 | 7.11E+05 | <1.0E-07 |

### Example 11: Stability test of humanized antibodies

### 1. Freeze and thaw

An appropriate amount of each antibody was taken and adjusted to 10 mg/mL, followed by freezing at -80°C for 10 min, and placing in an ice-water mixture to slowly thaw. The above procedure was repeated 5 times. Protein aggregation was detected using SEC-HPLC, and protein binding activity change was detected by ELISA.

### 2. Treatment at low pH

An appropriate amount of each antibody was taken and adjusted to 5 mg/mL in storage buffer adjusted to pH 3.6 using an ultrafiltration tube. The antibodies were placed at room temperature for 2-4 hours, then protein aggregation was detected using SEC-HPLC, and protein binding activity change was detected by ELISA.

### 3. 40°C acceleration test

An appropriate amount of each antibody was taken and adjusted to 10 mg/mL, and was placed at 40°C for 1 and 2 weeks. Protein aggregation was detected using SEC-HPLC, and protein binding activity change was detected by ELISA. The results were shown in Table 9.

**Table 9: stability test results of humanized antibodies**

| Antibody | Concentration (mg/ml) | SEC-HPLC | | | ELISA |
|---|---|---|---|---|---|
| | | HMW (%) | Main peak (%) | LW (%) | EC50 (µg/ml) |
| 53E5(hz) (Before treatment) | 10.457 | 5.5272 | 94.4728 | N | 0.05878 |
| 53E5(hz) (5 times of Freezing-thawing) | 10.568 | 4.3366 | 95.6635 | N | 0.06476 |
| 53E5(hz) (pH 3.6) | 5.004 | 4.2869 | 95.7131 | N | 0.06991 |
| 53E5(hz) (treated at 40°C for 1 week) | 10.897 | 5.3201 | 94.6799 | N | 0.06408 |
| 53E5(hz) (treated at 40°C for 2 weeks) | 10.784 | 5.3533 | 94.6467 | N | 0.05734 |
| | | | | | |

| Antibody | Concentration (mg/ml) | SEC-HPLC | | | ELISA |
|---|---|---|---|---|---|
| | | HMW (%) | Main peak (%) | LW (%) | EC50 (µg/ml) |
| 53A8(hz) (Before treatment) | 10.645 | 1.8110 | 98.1890 | | 0.04121 |
| 53A8(hz) (5 times of freezing-thawing) | 10.619 | 1.1731 | 98.8269 | | 0.06728 |
| 53A8(hz) (pH 3.6) | 5.351 | 1.3127 | 98.6873 | | 0.06905 |
| 53A8(hz) (treated at 40°C for 1 week) | 10.847 | 1.6034 | 98.3966 | | 0.06426 |
| 53A8(hz) (treated at 40°C for 2 weeks) | 10.925 | 1.4989 | 98.2745 | 0.2266 | 0.02964 |

### 4. Plasma stability test

200 µg of the antibodies to be tested (final concentration 1mg/ml) were added to 200µl of plasma and PBS, respectively, tightly sealed and placed at 37°C for 2 weeks. The change of protein binding activity was detected by ELISA. The results were shown in Table 10 and Figure 7.

**Table 10: ELISA results of humanized antibodies binding to human GDF15**

| Antibody | EC50 (µg/ml) |
|---|---|
| 53E5(hz) (PBS) | 0.1272 |
| 53E5(hz) (plasma) | 0.03725 |
| 53A8(hz) (PBS) | 0.1125 |
| 53A8(hz) (plasma) | 0.04129 |

### Example 12: Blocking the binding of GDF15 to the receptor GFRAL by humanized antibodies detected with ELISA

The extracellular region of GFRAL was stably expressed in the HEK293 luciferase reporter cell line, in which the expression of the luciferase gene was controlled by the SRE expression element, and the HEK293 SRE-LUC2-cRET-GFRAL reporter cell line was established.

### Blocking experiment 1:

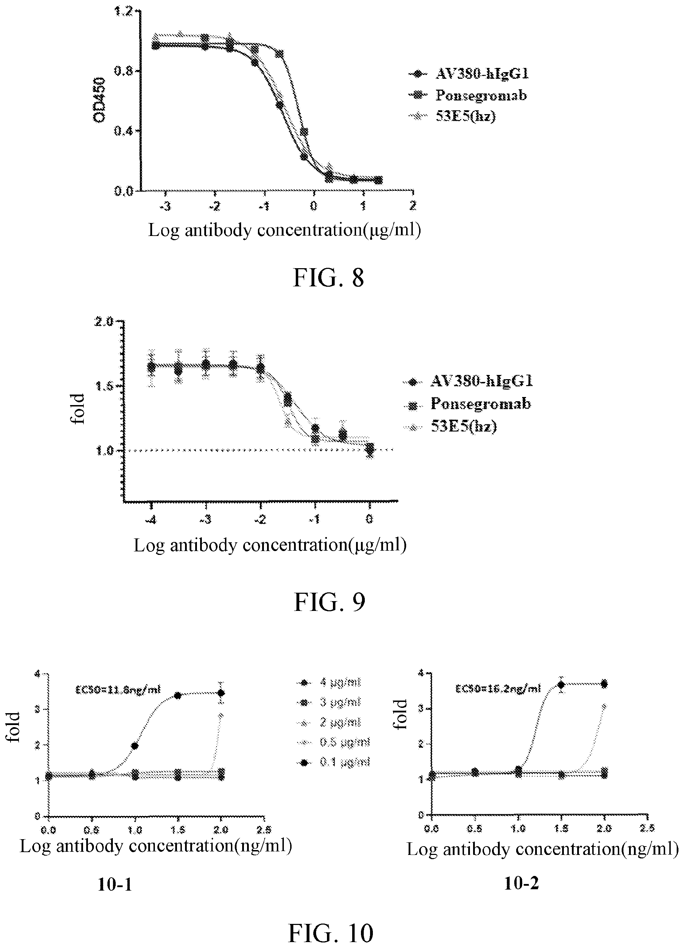

GFRAL-HIS protein (ACROBiosystems, GFA-H52H3) was dissolved in PBS at a concentration of 1 µg/ml and was used to coat ELISA 96-well plate one day before blocking the plate with 2% BSA in PBS. 53E5(hz), and control antibodies Ponsegromab and A V3 80-hIgG 1 were 3.16-fold diluted for 10 gradients with a starting concentration of 20µg/ml. At the same time, GDF15-mFc protein was added at a final concentration of 0.3µg/ml. After incubation at room temperature for 1 hour, the mixtures were added to 96-well plate coated with GFRAL-HIS. After incubated at room temperature for 1 hour, the plate was washed and then HRP-anti-mouse IgG was added to the plate. After incubation at room temperature for 1 hour, the HRP enzyme activity (OD450) was detected. The HRP enzyme activity represents the relative amount of GDF15-mFc protein bound to GFRAL. The OD450 values and antibody concentrations were plotted. The dose-effect curve was fitted using the log(agonist) vs. response-variable slope (four-parameter) Logistic model to calculate IC50. The experimental results shows that 53E5(hz) has a stronger ability of blocking GDF15 compared to the control antibodies. The results were shown in Table 11 and Figure 8.

**Table 11: competitive ELISA test results of humanized antibodies blocking the binding of GDF15 to its receptor GFRAL**

| Sample | IC50 (µg/ml) | OD450.Max |
|---|---|---|
| AV380-hIgG1 | 0.2311 | 0.965 |
| Ponsegromab | 0.5037 | 1.019 |
| 53E5(hz) | 0.2525 | 1.049 |

### Blocking experiment 2:

HEK293 SRE-LUC2-cRET-GFRAL reporter cells were cultured in 96-well plate. On the next day, 53E5(hz) and control antibodies Ponsegromab and AV380-hIgG1 were 3.16-fold diluted for 10 gradients with a starting concentration of 1µg/m. At the same time, GDF15-His (ACROBiosystems, GD5-H5149) was added at a final antibody concentration of 5ng/ml. After incubation at room temperature for 1 hour, the mixtures were added to the wells containing reporter cells to stimulate the luciferase expression. After culturing for 6 hours, 50µL of Bright-Glo reagent solution was added to each well, and the plate was shaken for 3-5 minutes to lyse the cells. The luminescence value was read using Multi-mode Microplate Reader. The luciferase activity increasing folds (enzyme activity folds of the wells without GDF15) and the antibody concentrations were plotted. The dose-effect curve was fitted using the log(agonist) vs. response-variable slope (four-parameter) Logistic model to calculate IC50. The experimental results show that 53E5(hz) and the control antibody Ponsegromab have similar inhibitory effects on GDF15. The results were shown in Table 12 and Figure 9.

**Table 12: results of humanized antibodies blocking GDF15 function on reporter cells expressing GFRAL**

| Sample | IC50 (µg/ml) | Bottom |
|---|---|---|
| 53E5(hz) | 0.02254 | 1.096 |
| AV380-hIgG1 | 0.04471 | 1.033 |
| Ponsegromab | 0.03211 | 1.066 |

### Blocking experiment 3:

HEK293 SRE-LUC2-cRET-GFRAL reporter cells were cultured in 96-well plate. On the next day, 53E5 (hz) and control antibody Ponsegromab were prepared as solutions at concentrations of 4, 3, 2, 0.5 and 0.1µg/ml respectively with culture medium. Five samples were taken for each antibody concentration and GDF15-His (ACROBiosystems, GD5-H5149) at different concentrations was added at a final concentrations of 100, 31.6, 10, 3.16 or 1ng/ml. After incubation at room temperature for 1 hour, the mixtures were added to the wells containing reporter cells to stimulate the luciferase expression. After culturing for 6 hours, 50µL of Bright-Glo reagent solution was added to each well, and the plate was shaken for 3-5 minutes to lyse the cells. The luminescence value was read using Multi-mode Microplate Reader. The luciferase activity increasing folds (enzyme activity folds of the wells without GDF15 protein) and antibody concentrations were plotted. The dose-effect curve was fitted using the log(agonist) vs. response-variable slope (four-parameter) Logistic model to calculate EC50.

The experimental results show that 53E5(hz) has stronger effects of neutralizing GDF15 compared to control antibody Ponsegromab. The results were shown in Fig. 10-1 and 10-2. In this experiment, the antibodies at a fixed concentration were co-incubated with GDF15 protein at different concentrations, and the signals of the reporter cells with non-neutralized GDF15 stimulation were detected. The EC50 shown in the figure is the value of the antibodies at 0.1 µg/ml.

### Example 13: Detection of binding specificity of humanized antibodies

### Binding specificity experiment 1:

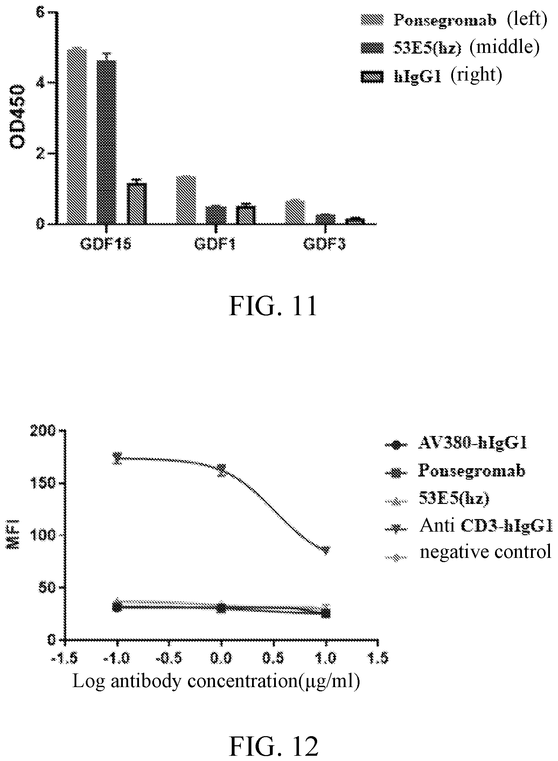

GDF15 (ACROBiosystems, GD5-H5149), GDF1 (R&D, 6937-GD-010), and GDF3 (R&D, 5754-G3-010) proteins were dissolved in PBS at a concentration of 1 µg/ml and were used to coat ELISA 96-well plate. On the next day, the 96-well plate was blocked with 2% BSA in PBS for 1 hour. After removing the blocking solution, GDF15 antibody 53E5 (hz), control antibody Ponsegromab and irrelevant hIgG1 were added at 4µg/ml. After incubation at room temperature for 1 hour, the plate was washed, and then HRP-anti-human IgG was added. After incubation at room temperature for 1 hour, the HRP enzyme activity signal (OD450) was detected. The OD450 value is the relative signal of the antibody binding amount. The experimental results show that the antibody 53E5 (hz) has no specific binding to GDF1 and GDF3, while the control antibody Ponsegromab has weak binding to GDF1 and GDF3. The results were shown in Figure 11.

### Binding specificity experiment 2:

Frozen PBMC was thawed followed by adding 10, 1, or 0.1µg/ml of GDF15 antibody 53E5 (hz), control antibodies Ponsegromab and AV380-hIgG1, or CD3 antibody OKT3 as a positive control. A tube without antibody was taken as a negative control. After incubation at 4°C for 30 minutes, the antibodies were removed, and PE anti-human IgG flow analysis antibody was added. After incubation at 4°C for 30 minutes, the cells were spun down and antibody diluent was added. 7 AAD was added to remove dead cells. The antibody binding signal was analyzed by flow cytometry. On PBMC, the mean fluorescence intensity (MFI) of PE represents the signal of antibodies binding to cells. The antibody concentrations and MFI were plotted. The experimental results show that antibody 53E5 (hz) has no specific binding to PBMC. The results were shown in Figure 12.

### Example 14: In vivo PK experiment in animals

10-12 week old, femal BALB/c mice were injected via tail vein with GDF15 antibody 53E5 (hz), control antibodies AV380-hIgG1 and Ponsegromab, at doses of 10 mg/kg and 1 mg/kg mouse body weight. Venous blood was collected at 10 mins, 1 hour, 6 hours, 1 day, 3 days, 5 days, 7 days, and 14 days to obtain serum. After collecting serum at all the time points, the antibody concentrations in the serum was detected by ELISA.

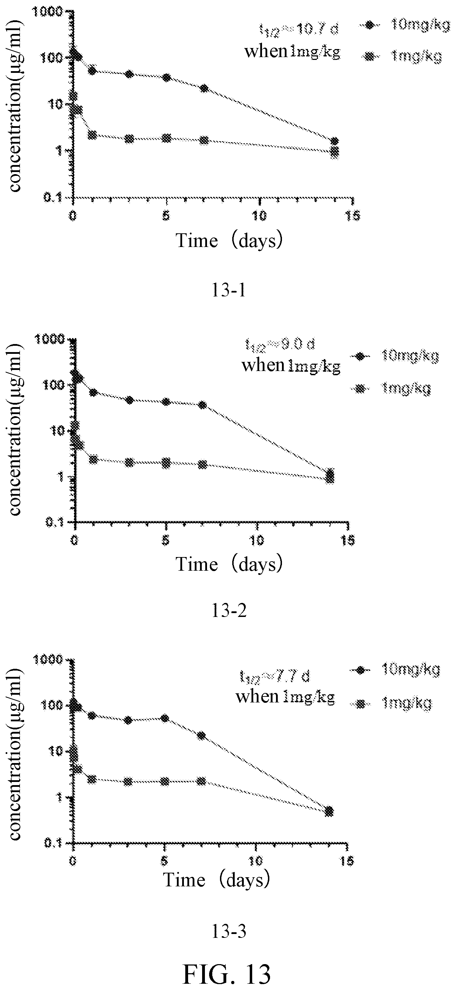

The process is as follows: the plate was coated with GDF15-HIS antigen overnight at 4°C, 50 ng per well, and was blocked with 2% BSA blocking solution for 2 hours. The serum samples were diluted 50-fold and 500-fold; the standard (i.e., the injected antibody) was starting at 1µg/mL, 3.16-fold dilution for 12 gradients. The blocking solution was removed, and the prepared serum samples and the standards were added, and incubated at room temperature for 1 hour. After washing, HRP-anti-human IgG was added and the plate was incubated at room temperature for 1 hour. After washing, the HRP enzyme activity (OD450) was detected. The OD450 values and standard concentrations were plotted, and the antibody concentrations in the serum samples were calculated based on the fitted standard curve. The blood collection times and the antibody concentrations in the serum were plotted, and the half-life of the antibody in mice was calculated. The results were shown in Figure 13.

### Example 15: In vivo functional assay of GDF15 antibody

### Experiment 1. Testing the efficiency of GDF15 antibodies in inhibiting cachexia.

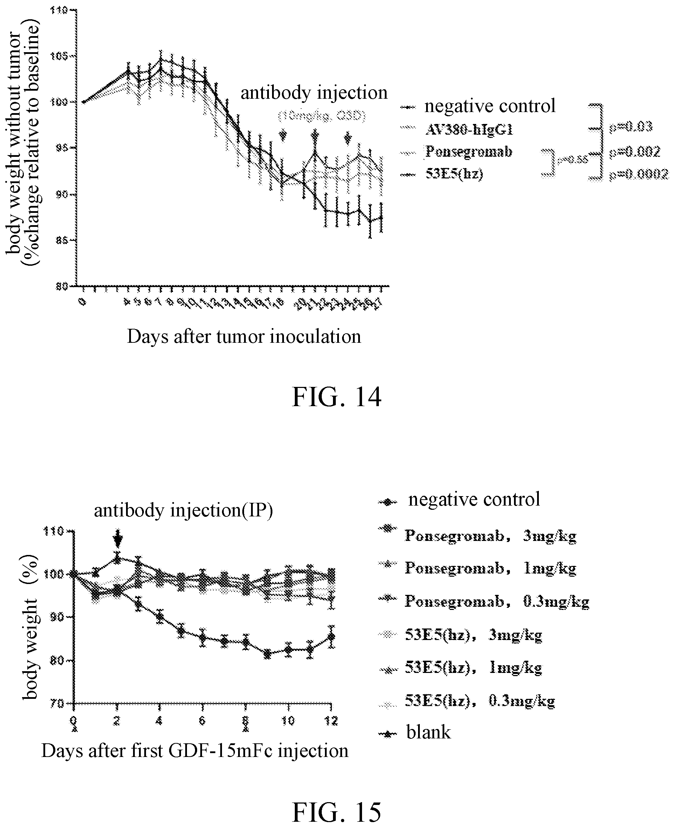

8-10 week old, female SCID mice were injected subcutaneously with 5×10⁶ HT1080 cells (containing 50% matrigel), body weights were measured every day, and tumor sizes (length and width of the tumor) were also measured every day with a caliper to calculate tumor volume (=0.5x length × width × height). The weight of mice without tumor was the weight of mice with tumor minus the tumor weight (the tumor weight was calculated as volume*1mg/mm³). When the mice body weight dropped to 93% of the weight on day 0 (which is day 18 in this experiment), the mice were randomly divided into groups, with 10 mice in each group, and intraperitoneally injected at a dose of 10 mg/kg mouse body weight with hIgG (negative control) or humanized antibody 53E5(hz) or control antibodies AV380-hIgG1 and Ponsegromab once every 3 days. The weights of mice without tumor and the days after tumor inoculation were plotted, and the results were shown in Figure 14. The experimental results showed that 53E5(hz) and the control antibody Ponsegromab had similar effects in inhibiting cachexia.

### Experiment 2. Testing the efficiency of GDF-15 antibodies in neutralizing GDF15 in vivo.

BALB/c mice were intraperitoneally injected (I.P.) with GDF15-mFc fusion protein (0.25 mg/kg body weight) on day 0 and day 8, and hIgG (3 mg/kg body weight, negative control) or different doses of humanized antibody 53E5(hz) and control antibody Ponsegromab (3, 1, 0.3 mg/kg body weight) were injected on the next day. The body weights were measured every day.

At the end of the experiment, the mouse body weights and the days after injection of GDF15-mFc fusion protein were plotted, and the results were shown in Figure 15. The experimental results showed that 53E5(hz) and the control antibody Ponsegromab had similar effects in inhibiting GDF15 function *in vivo.*

The above description of the specific embodiments of the present disclosure does not limit the present invenion. Those skilled in the art would make various changes or modifications based on the present disclosure, as long as they do not depart from the spirit of the present disclosure, they should all fall within the scope of the claims of the present disclosure.

## Claims

1. An antibody or fragment thereof, comprising a variable region of a heavy chain (VH) and a variable region of a light chain (VL), wherein the variable region of the heavy chain (VH) and the variable region of the light chain (VL) comprise a combination of H-CDR1, H-CDR2, H-CDR3, L-CDR1, L-CDR2 and L-CDR3 selected from:
(1) H-CDR1, H-CDR2 and H-CDR3 set forth in SEQ ID NO.23 (TSGMGVG), SEQ ID NO.24 (HII,WDDVKRYNPALKS) and SEQ ID NO.25 (MAWDWFAY), respectively, and L-CDR1, L-CDR2 and L-CDR3 set forth in SEQ ID NO.26 (KASQNVDTNVA), SEQ ID NO.27 (SASYRSS) and SEQ ID NO.28 (QQYHSYPT), respectively;
(2) H-CDR1, H-CDR2 and H-CDR3 set forth in SEQ ID NO.23 (TSGMGVG), SEQ ID NO.29 (HIRWDDVKRYNPALKS) and SEQ ID NO.25 (MAWDWFAY), respectively, and L-CDR1, L-CDR2 and L-CDR3 set forth in SEQ ID NO.30 (KASQNVDTDVA), SEQ ID NO.31 (SASYRYS) and SEQ ID NO.32 (HQYNSYPT), respectively;
(3) H-CDR1, H-CDR2 and H-CDR3 set forth in SEQ ID NO.33 (TAGMTVG), SEQ ID NO.34 (HIWWNDDKYYNPALKS) and SEQ ID NO.35 (IATMNYAMDY), respectively, and L-CDR1, L-CDR2 and L-CDR3 set forth in SEQ ID NO.36 (RASQSVSTSSFSYMH), SEQ ID NO.37 (YASNLES) and SEQ ID NO.38 (QHSWEIPYT), respectively;
(4) H-CDR1, H-CDR2 and H-CDR3 set forth in SEQ ID NO.39 (TSGMGVD), SEQ ID NO.40 (HIYWDDDKRYNPSLKS) and SEQ ID NO.41 (RAWDAMDY), respectively, and L-CDR1, L-CDR2 and L-CDR3 set forth in SEQ ID NO.42 (KARQNVGTNVA), SEQ ID NO.31 (SASYRYS) and SEQ ID NO.43 (QQYNSYPYT), respectively;
(5) H-CDR1, H-CDR2 and H-CDR3 set forth in SEQ ID NO.23 (TSGMGVG), SEQ ID NO.44 (HIWWNDDKYYNPSLKS) and SEQ ID NO.45 (GAYDFFDY), respectively, and, L-CDR1, L-CDR2 and L-CDR3 set forth in SEQ ID NO.26 (KASQNVDTNVA), SEQ ID NO.31 (SASYRYS) and SEQ ID NO.46 (QQYNTYPYT), respectively; and
(6) H-CDR1, H-CDR2 and H-CDR3 set forth in SEQ ID NO.47 (TSGIGIT), SEQ ID NO.48 (TIWWDDDNRYNPSLKS) and SEQ ID NO.49 (SAWDWFAY), respectively, and L-CDR1, L-CDR2 and L-CDR3 set forth in SEQ ID NO.50 (KASQNVGTNVA), SEQ ID NO.51 (SASYRNS) and SEQ ID NO. 52 (QQYNSHPVT), respectively.

2. The antibody or fragment thereof according to claim 1, wherein the variable region of the heavy chain comprises an amino acid sequence set forth in or an amino acid sequence having at least 75% identity with SEQ ID NO: 7, SEQ ID NO: 9, SEQ ID NO: 11, SEQ ID NO: 13, SEQ ID NO: 15, SEQ ID NO: 17, SEQ ID NO: 19 or SEQ ID NO: 21; and/or the variable region of the light chain comprises an amino acid sequence set forth in or an amino acid sequence having at least 75% identity with SEQ ID NO: 8, SEQ ID NO: 10, SEQ ID NO: 12, SEQ ID NO: 14, SEQ ID NO: 16, SEQ ID NO: 18, SEQ ID NO: 20 or SEQ ID NO: 22.

3. The antibody or fragment thereof according to claim 1 or 2, wherein the variable region of the heavy chain and the variable region of the light chain respectively comprise:
(1) an amino acid sequence set forth in or an amino acid sequence having at least 75% identity with SEQ ID NO: 7, and an amino acid sequence set forth in or an amino acid sequence having at least 75% identity with SEQ ID NO: 8;
(2) an amino acid sequence set forth in SEQ ID NO: 9 or an amino acid sequence having at least 75% identity thereto, and an amino acid sequence set forth in or an amino acid sequence having at least 75% identity with SEQ ID NO: 10;
(3) an amino acid sequence set forth in or an amino acid sequence having at least 75% identity with SEQ ID NO: 11, and an amino acid sequence set forth in or an amino acid sequence having at least 75% identity with SEQ ID NO: 12;
(4) an amino acid sequence set forth in or an amino acid sequence having at least 75% identity with SEQ ID NO: 13; and, an amino acid sequence set forth in or an amino acid sequence having at least 75% identity with SEQ ID NO: 14;
(5) an amino acid sequence set forth in or an amino acid sequence having at least 75% identity with SEQ ID NO: 15, and an amino acid sequence set forth in or an amino acid sequence having at least 75% identity with SEQ ID NO: 16;
(6) an amino acid sequence set forth in or an amino acid sequence having at least 75% identity with SEQ ID NO: 17, and an amino acid sequence set forth in or an amino acid sequence having at least 75% identity with SEQ ID NO: 18;
(7) an amino acid sequence set forth in or an amino acid sequence having at least 75% identity with SEQ ID NO: 19, and an amino acid sequence set forth in or an amino acid sequence having at least 75% identity with SEQ ID NO: 20;
(8) an amino acid sequence set forth in or an amino acid sequence having at least 75% identity with SEQ ID NO: 21, and an amino acid sequence set forth in or an amino acid sequence having at least 75% identity with SEQ ID NO: 22.

4. The antibody or fragment thereof according to any one of claims 1 to 3, wherein the antibody is a murine antibody, a chimeric antibody or a fully or partially humanized antibody; the antigen-binding fragment is a semi-antibody or a scFv, dsFv, (dsFv)₂, Fab, Fab', F(ab')₂ or Fv fragment thereof;
preferably, the antibody is a monoclonal antibody or a single-chain antibody;
preferably, the antibody or fragment thereof further comprises a human or murine constant region, preferably a human or murine constant region of a heavy chain (CH) and/or a human or murine constant region of a light chain (CL); preferably, the antibody or fragment thereof comprises a heavy chain and a light chain; more preferably, the antibody or fragment thereof comprises a constant region of a heavy chain selected from IgG, IgA, IgM, IgD or IgE and/or a constant region of a kappa or lambda light chain.

5. The antibody or fragment thereof according to any one of claims 1 to 4, wherein the antibody is a monoclonal antibody, preferably a murine, chimeric or humanized monoclonal antibody; more preferably, the monoclonal antibody is IgG, specifically IgG1.

6. A nucleic acid molecule comprising a nucleotide sequence encoding a CDR of a heavy chain, a CDR of a light chain, a variable region of a light chain, a variable region of a heavy chain, a heavy chain or a light chain comprised in the antibody or fragment thereof according to any one of claims 1 to 5.

7. A vector comprising the nucleic acid molecule of claim 6.

8. A host cell comprising the nucleic acid molecule of claim 6 and/or the vector of claim 7.

9. A composition comprising the antibody or fragment thereof according to any one of claims 1 to 5, the nucleic acid molecule according to claim 6, the vector according to claim 7 and/or the host cell according to claim 8;
preferably, the composition is a pharmaceutical composition, which optionally comprises a pharmaceutically acceptable excipient.

10. Use of the antibody or fragment thereof according to any one of claims 1 to 5, the nucleic acid molecule according to claim 6, the vector according to claim 7, the host cell according to claim 8 and/or the composition according to claim 9 in the manufacture of a medicament for treating a disease or condition mediated by or associated with the expression of GDF15;
and/or, the disease or disorder is tumor (e.g., cancer), heart failure, chronic kidney disease, anorexia, sarcopenia, or cachexia.

11. A method for treating a disease or condition, comprising administering to a subject in need thereof the antibody or fragment thereof of any one of claims 1 to 5, the nucleic acid molecule of claim 6, the vector of claim 7, the host cell of claim 8 and/or the composition of claim 9, wherein the disease or condition is mediated by or associated with the expression of GDF15; and/or the disease or condition is tumor (e.g., cancer), heart failure, chronic kidney disease, anorexia, sarcopenia or cachexia;
preferably, the subject is a mammal; more preferably, the subject is a human.

12. A method for detecting or diagnosing a disease or condition, comprising contacting the antibody or fragment thereof according to any one of claims 1 to 5, the nucleic acid molecule according to claim 6, the vector according to claim 7, the host cell according to claim 8 and/or the composition according to claim 9 with a sample from a subject, wherein the disease or condition is mediated by or associated with the expression of GDF15; or, the disease or condition is tumor (e.g., cancer), heart failure, chronic kidney disease, anorexia, sarcopenia or cachexia;
preferably, the subject is a mammal; more preferably, the subject is a human.

13. A kit comprising the antibody or fragment thereof according to any one of claims 1 to 5, the nucleic acid molecule according to claim 6, the vector according to claim 7, the host cell according to claim 8 and/or the composition according to claim 9;
optionally, the kit further comprises instructions for use.
